(19) Europäisches Patentamt
European Patent Office
Office européen des brevets

(11) Veröffentlichungsnummer: **0 063 779**
**A1**

(12) # EUROPÄISCHE PATENTANMELDUNG

(21) Anmeldenummer: **82103330.5**

(22) Anmeldetag: **21.04.82**

(51) Int. Cl.³: **C 07 D 307/935**, A 61 K 31/34

(30) Priorität: **22.04.81 HU 103581**

(43) Veröffentlichungstag der Anmeldung: **03.11.82**
**Patentblatt 82/44**

(84) Benannte Vertragsstaaten: **BE CH DE FR GB IT LI NL SE**

(71) Anmelder: **CHINOIN Gyógyszer és Vegyészeti Termékek Gyára RT., Tó utca 1-5, H-1045 Budapest V (HU)**

(72) Erfinder: **Szántay, Csaba, Dr. Dipl.-Ing.-Chem., Zsombolyai u. 8, H-1113 Budapest (HU)**
Erfinder: **Novák, Lajos, Dr. Dipl.-Ing.-Chem., Szolyva u. 2/b, H-1126 Budapest (HU)**
Erfinder: **Aszódi, József, Dipl.-Ing.-Chem., Stromfeld u. 25, H-2132 Gög-felsö (HU)**
Erfinder: **Simonidesz, Vilmos, Dr. Dipl.-Ing.-Chem., Fenyö u. 3, H-1016 Budapest (HU)**
Erfinder: **Galambos, Géza, Dipl.-Ing.-Chem., Lavotta u. 60, H-1104 Budapest (HU)**
Erfinder: **Kovács, Gábor, Dr. Dipl.-Ing.-Chem., Rona Park 4, H-1142 Budapest (HU)**
Erfinder: **Virág, Sándor, Dr., Sallai I. u. 29/a, H-1136 Budapest (HU)**
Erfinder: **Stadler, István, Dr., Népstadion u. 18, H-1143 Budapest (HU)**
Erfinder: **Körmoczy, Péter, Dr., Uri u. 33, H-1014 Budapest (HU)**

(74) Vertreter: **Lotterhos, Hans Walter, Dr.-Ing., Lichtensteinstrasse 3, D-6000 Frankfurt am Main 1 (DE)**

(54) **13-Thia-prostacyclin-Derivate, Verfahren zu deren Herstellung sowie diese Verbindungen enthaltende pharmazeutische Präparate.**

(57) Die Erfindung betrifft recemische oder optisch aktive 13-Thia-prostacyclin-Derivate der Formel I

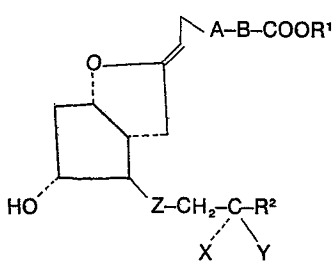

(I)

($R^1$ = H, pharmazeutisch verträgliches Kation, geradkettiges oder verzweigtes $C_{1-5}$-Alkyl,
$R^2$ = geradkettiges oder verzweigtes $C_{4-9}$-Alkyl oder monosubstituiertes Phenoxymethyl,
$-A-B-$ = $-CH_2-CH_2-$, Z- oder E- $-CH=CH-$ oder $-C\equiv C-$, eines der Symbole X und Y = H, Methyl oder Äthyl und das andere = Hydroxy, Tetrahydropyranyloxy, 1-Äthoxy-äthoxy oder Tri-$C_{1-5}$-alkylsilyloxy oder
X und Y gemeinsam $= -O-CH_2-CH_2-O-$ und
Z = $-S-$ oder $-SO-$)
sowie deren Herstellung aus 13-Thia-prostaglandin-Derivaten der Formel III

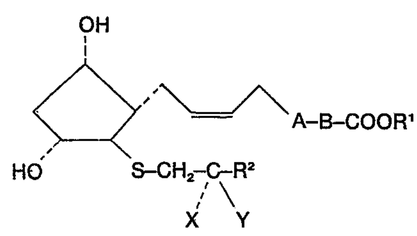

(III),

die – gegebenenfalls nach Oxydation zum 13-Oxyd-Derivat – durch Umsetzung mit einem Halogenierungsmittel in Gegenwart einer Base zu den 5-Halogen-13-thia-prostacyclin-Derivaten der Formel II

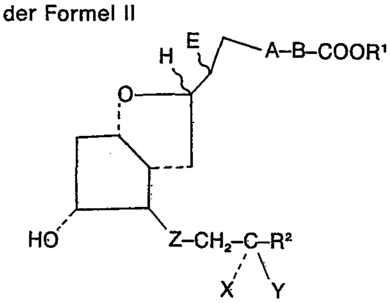

(II)

(Fortsetzung nächste Seite)

EP 0 063 779 A1

ACTORUM AG

cyclisiert und durch Behandeln mit einer organischen oder anorganischen Base dehydrohalogeniert werden.

Die Verbindungen der Formel I hemmen die Blutplättchenaggregation, üben eine Kontraktionswirkung auf die Trachea aus und können in der Therapie verwendet werden.

# 13-Thia-prostacyclin-Derivate, Verfahren zu deren Herstellung sowie diese Verbindungen enthaltende pharmazeutische Präparate

Die Erfindung betrifft neue, optisch aktive und racemische 13-Thia-prostacyclin-Derivate der allgemeinen Formel I

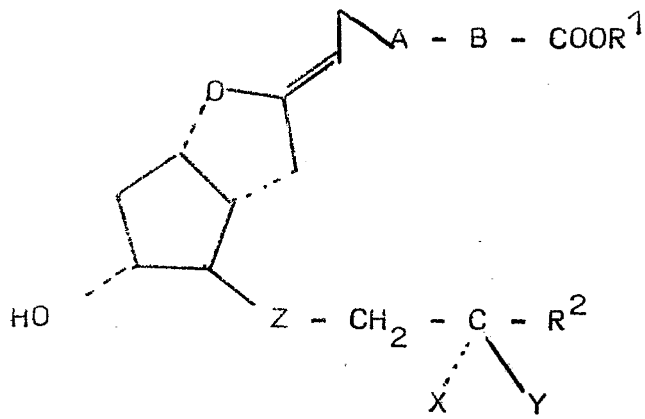

(I) ,

worin

$R^1$ Wasserstoff, ein Äquivalent eines pharmazeutisch verträglichen Kations oder geradkettiges oder verzweigtes $C_{1-5}$-Alkyl,

$R^2$ geradkettiges oder verzweigtes $C_{4-9}$-Alkyl oder monosubstituiertes Phenoxymethyl,

-A-B- $-CH_2-CH_2-$, Z- oder E- -CH=CH-, oder -C≡C-,

eines der Symbole X und Y Wasserstoff, Methyl oder Äthyl und das andere Hydroxy, Tetrahydropyranyloxy, 1-Äthoxy-äthoxy oder Tri-$C_{1-5}$-alkylsilyloxy bedeuten oder

X und Y gemeinsam $-O-CH_2-CH_2-O-$ bilden und

Z Schwefel oder -SO- ist.

Die biologisch aktiven neuen Verbindungen der allgemeinen Formel I können als stabilisierte Analoga des biologisch äußerst wirksamen Prostacyclins angesehen werden. Das Prostacyclin wurde zum ersten Mal im Jahre 1976 beschrieben (S.Moncada und Mitarbeiter: Nature 263, 663). Die Struktur dieser Verbindung wurde bald aufgeklärt (R.A. Johnson und Mitarbeiter: Prostaglandines, 12, 915 (1976)). Das Prostacyclin ist ein Zwischenprodukt des Arachidonsäuremetabolismus und übt bereits in einer sehr geringen Menge äußerst starke und wertvolle pahrmazeutische Wirkungen aus. Von diesen Wirkungen scheint die die Blutplättchenaggregation hemmende und die vasodilatatorische Wirkung am wichtigsten zu sein. Mit Hilfe dieser Wirkungen kann das Prostacyclin als Arzneimittel zur Verhütung und Behandlung der heutzutage so häufigen Kreislaufanomalien - wie die Arteriosklerose und Thrombose - Verwendung finden. In der Fachliteratur sind über die klinische Anwendung des Prostacyclins mehrere Artikel erschienen (z.B. J.R.Vane und S.Bergström: Prostacyclin, Raven Press, New York 1979).

Das wichtigste Hindernis bei der praktischen Anwendung des Prostacyclins stellt dessen äußerst große Instabilität dar. Obwohl der chemisch empfindlichste Punkt des Prostacyclinmoleküls die Enolätherstruktur ($C_{5-9}$) ist - in Gegenwart einer äußeren oder inneren Protonenquelle zersetzt sich das Molekül schnell unter Bildung von 6-Oxo-PGF$_{2\alpha}$ - ist dessen Metabolismus außergewöhnlich schnell. Im Organismus wird das Prostacyclin durch das Prostaglandindehydrogenase-enzymsystem unter Oxydation der $C_{13-20}$ Struktureinheit rasch desaktiviert. Das Prostacyclin ist in Form der freien Säure instabil; zu den pharmakologischen und klinischen Untersuchungen werden dessen Salze und Ester verwendet.

Ein Weg zur Stabilisierung des Prostacyclins besteht in der Durchführung von strukturellen Änderungen am Molekül, die die Wirkung des Prostaglandindehydrogenase-enzymsystems herabsetzen.

Die neuen Verbindungen der allgemeinen Formel I der Erfindung enthalten an Stelle der 13,14-C-C-Doppelbindung ein Schwefelatom und eine Methylengruppe oder eine -SO-Gruppe und eine Methylengruppe. Mit Rücksich darauf, daß der Angriffspunkt des Dehydrogenase-enzymsystems die Stellung 15 und 13 - 14 ist, kann wegen der erfindungsgemäßen Änderung das Molekül nur in geringerem Maße ein Substrat des Prostacyclin-dehydrogenase-enzymsystems sein, wobei die wertvolle pharmakologische Wirksamkeit des Prostacyclins erhalten bleibt.

0063779

Die neuen Verbindungen der Formel I hemmen an aus männlichem Blut isoliertem, an Blutplättchen reichem Plasma die durch 1 x $10^{-6}$ Mol/ml ADP erzeugte Aggregation in einer Konzentration von 50 - 100 mg/ml ($ID_{50}$) - nach der Methode von Born gemessen - was einer 1/10 - 1/50 $PGI_2$-Wirkung entspricht.

An aus Kaninchenblut isoliertem, an Blutplättchen reichem Plasma kann eine 50 %ige Hemmung der durch 1 x $10^{-4}$ Mol/ml ADP verursachten Aggregation
bei Anwendung der Verbindungen der Formel I in Form der Ester mit einer Konzentration von 1 - 10 g/ml und
bei Anwendung in Form der Salze mit einer Konzentration von 500 - 900 ng/ml erreicht werden.

Die Wirkung, die die Verbindungen der allgemeinen Formel I auf den Blutdruck von mit Pentobarbitural narkotisierten Katzen ausüben, entspricht etwa der Wirkung von 1/100 $PGI_2$, d.h. sie ist um zwei Größenordnungen kleiner als die von $PGI_2$.

Sowohl die Aggregations-hemmende als auch die blutdrucksenkende Wirkung weisen auf einen Zusammenhang zwischen Dosis und Wirkung hin.

In ähnlicher Weise wie das $PGI_2$ zeigen die Verbindungen der allgemeinen Formel I an der Meerschweinchentrachea eine Kontraktionswirkung. Die Aktivität ist etwa um eine Größenordnung schwächer als die von $PGI_2$.

Die Erfindung betrifft auch ein Verfahren zur Herstellung der Verbindungen der allgemeinen Formel I

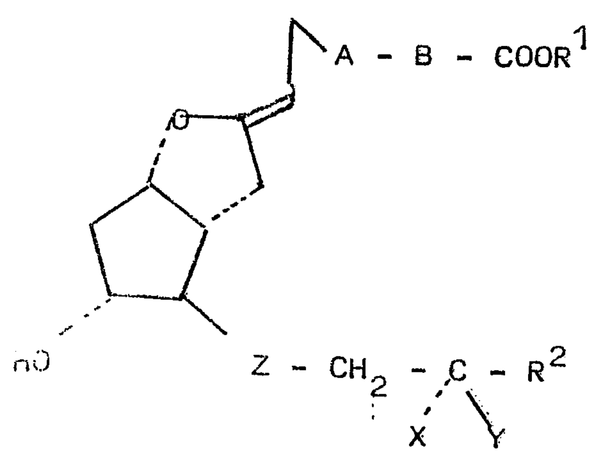

(I) ,

worin $R^1$, $R^2$, -A-B-, X, Y und Z die oben angegebene Bedeutung haben, wonach man ein 13-Thia-prostaglandin-Derivat der allgemeinen Formel III

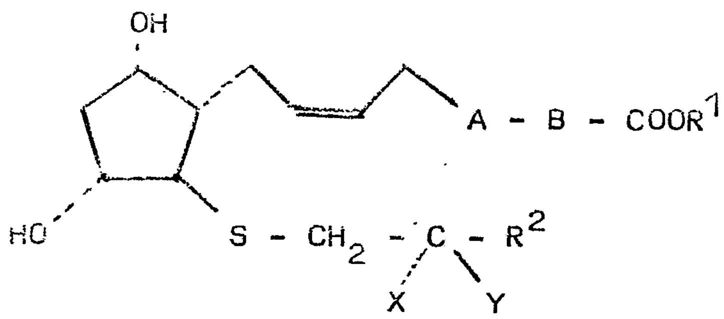

(III),

worin $R^1$, $R^2$, -A-B-, X und Y die oben angegebene Bedeutung haben, - gewünschtenfalls nach Oxydation zum entsprechenden 12-Oxyd-Derivat - in Gegenwart einer Base mit einem Halogenierungsmittel umsetzt, das erhaltene 5-Halogen-13-thia-prostacyclin-Derivat der allgemeinen Formel II

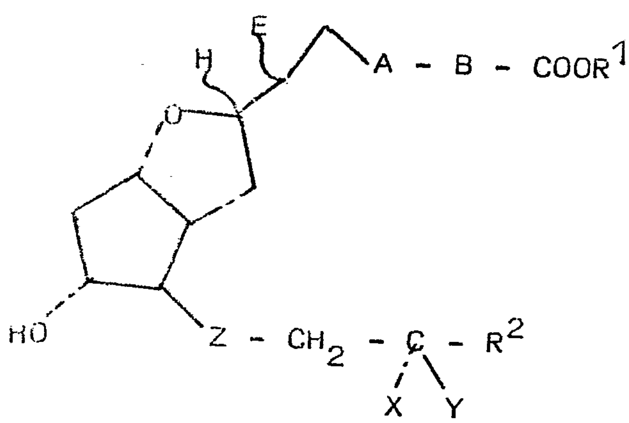

(II),

worin $R^1$, $R^2$, -A-B-, X, Y und Z die oben angegebene Bedeutung haben, und E Halogen ist - falls E Brom ist, nach Abtrennung des Endo-Isomers bzw. falls E Jod ist, in Form des Isomerengemisches - mit einer organischen oder anorganischen Base behandelt und das erhaltene 13-Thia-prostacyclin-Derivat der allgemeinen Formel I gewünschtenfalls - wenn X und/oder Y eine Schutzgruppe tragen - durch Hydrolyse von der Schutzgruppe befreit und/oder in ein Salz überführt oder aus einem Salz freisetzt.

Die Cyclisierung kann mit Hilfe eines Halogenierungsmittels - vorzugsweise Jod - durchgeführt werden. Die Umsetzung wird in Gegenwart einer nicht zu starken Base - zweckmäßig Kaliumcarbonat oder Natriumbicarbonat - durchgeführt. Die Reaktionstemperatur liegt zweckmäßig zwischen etwa 0 und 30°C. Die Halogenierung wird in einem mäßig polaren aprotischen Lösungsmittel durchgeführt. Als Reaktionsmedium können organische Lösungsmittel - insbesondere Dichlormethan oder Chloroform - verwendet werden. Das aus dem Reaktionsgemisch durch Extraktion isolierte Rohprodukt kann durch Chromatographie gereinigt werden.

Bei der Anwendung von Jod entsteht das Exo-Isomer der allgemeinen Formel IIa

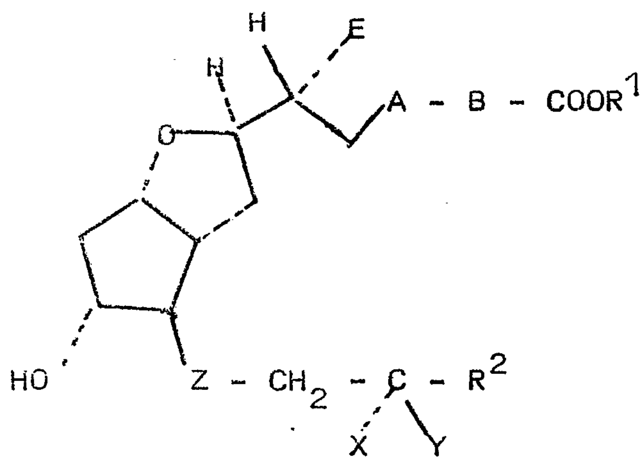

(IIa)

in einer Menge von mehr als 95 %, wobei das in einer Menge von etwa 4 % gebildete Endo-Isomer der allgemeinen Formel IIb

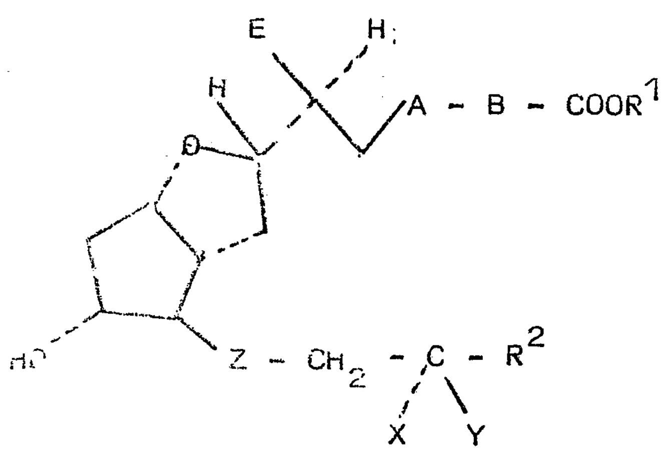

(IIb)

nicht abgetrennt werden muß.

Bei Anwendung von Bromierungsmitteln beträgt die Menge des gebildeten Endo-Isomers der allgemeinen Formel IIb etwa 10 %. In dem Fall wird das Endo-Isomer durch Säulenchromatographie abgetrennt, und zur Herstellung der Prostacyclin-Derivate der allgemeinen Formel I werden die Exo-Isomere verwendet.

Ein Teil der als Ausgangsverbindung verwendeten Verbindungen der allgemeinen Formel III ist aus der BE-PS 828 925 bekannt. Die dort nicht beschriebenen 13-Thia-prostaglandin-13-oxyde, die anstelle von Z eine -SO-Gruppe enthalten, können aus den entsprechenden bekannten 13-Thia-prostaglandin-Derivaten, die anstelle von Z ein Schwefelatom enthalten, mit einem milden Oxydationsmittel, z.B. Natriumperjodat, in wäßrig-alkoholischem Medium - zweckmäßig in einem Gemisch von Wasser und Methanol - hergestellt werden.

Die Dehydrohalogenierung der 5-Halogen-13-thia-prostacyclin-Derivate der allgemeinen Formel II (worin $R^1$, $R^2$, -A-B-, X, Y und Z die oben angegebene Bedeutung haben und E Halogen, vorzugsweise Brom oder Jod ist) kann mit einer organischen oder anorganischen Base, vorteilhaft mit 1,5-Diazabicyclo[4.3.0]non-5-en - in einem wasserfreien aprotischen Lösungsmittel - zweckmäßig in Toluol oder Alkohol - oder in Abwesenheit eines Lösungsmittels durchgeführt werden. Die Reaktionstemperatur liegt im Bereich von 25 und 150°C, vorzugsweise von 100 - 120°C.

Die aus dem Reaktionsgemisch durch Waschen, Trocknen und Abdestillieren des Lösungsmittels isolierten Verbindungen der allgemeinen Formel I bedürfen im allgemeinen keiner weiteren Reinigung. Sollte eine Reinigung trotzdem notwendig sein, kann diese - mit Ausnahme der freien Säuren - durch Säulenchromatographie erfolgen.

Zur Herstellung von Verbindungen der allgemeinen Formel I, in welchen $R^1$ Wasserstoff oder ein Äquivalent eines pharmazeutisch verträglichen Kations bedeutet und $R^2$, -A-B-, X, Y und Z die oben angegebene Bedeutung haben, aus den entsprechenden $C_{1-5}$-Alkylestern wird zweckmäßig

so vorgegangen, daß man den erhaltenen $C_{1-5}$-Alkylester mit einer wäßrigen Alkalimetallhydroxydlösung verseift und mit einer Säure behandelt und gewünschtenfalls die so erhaltene Carbonsäure der allgemeinen Formel I in ein Salz mit einem pharmazeutisch verträglichen Kation überführt.

Die Bezeichnung "pharmazeutische verträgliches Kation" bezieht sich auf ein Äquivalent solcher ein-, zwei- oder dreiwertigen Kationen, die im lebenden Organismus in den den erfindungsgemäßen Verbindungen entsprechenden Dosen keine unerwünschten Nebenwirkungen hervorrufen. Einige Beispiele für pharmazeutisch verträgliche Kationen sind: Alkalimetallkationen (z.B. Natrium, Kalium, Lithium), Erdalkalimetallkationen (z.B. Calcium, Magnesium), Aluminiumkation, Ammoniumkation oder aus verschiedenen organischen Aminen ableitbare ein- oder mehrwertige Ammoniumionen (z.B. tris-(Hydroxymethyl)-ammonium-ion).

Unter der Bezeichnung "geradkettiges oder verzweigtes $C_{1-5}$-Alkyl" sind Methyl, Äthyl, n-Propyl, Isopropyl, n-Butyl, Isobutyl, sec. Butyl und tert. Butyl sowie die verschiedenen Amylgruppen zu verstehen und unter "geradkettiges oder verzweigtes $C_{4-9}$-Alkyl" die verschiedenen Butyl-, Amyl-, Hexyl-, Heptyl-, Octyl- und Nonylgruppen.

Der Phenylring der Phenyloxymethylgruppe kann in irgendeiner Stellung Halogen oder Trifluormethyl tragen.

Die drei $C_{1-5}$-Alkylsubstituenten der Trialkylsilylgruppe können identisch oder verschieden sein und die für die $C_{1-5}$-Alkylgruppen oben angegebene Bedeutung haben.

Unter dem Ausdruck "organische Base" sind ein oder mehrere Stickstoffatome enthaltende, lineare, verzweigte oder cyclische Alkylamine zu verstehen (vorteilhaft 1,5-Diazabicyclo[4.3.0]non-5-en oder Triäthylamin). Die "anorganischen Basen" können aus Alkalimetallen oder Erdalkalimetallen herleitbar sein, wie z.B. Alkoholate, Hydroxyde, Carbonate, Bicarbonate usw..

Die bei dem Verfahren der Erfindung verwendbaren Alkohole enthalten vorzugsweise 1 - 5 Kohlenstoffatome. Als aprotische Lösungsmittel können vorteilhaft aromatische Kohlenwasserstoffe (wie Benzol, Toluol) oder halogenierte Kohlenwasserstoffe (wie Chloroform, Dichlormethan usw.) eingesetzt werden.

Als Halogenierungsmittel werden in erster Linie elementare Halogene (wie Jod oder Brom) benutzt. Es können jedoch auch andere aus der organischen Chemie bekannten Halogenierungsmittel eingesetzt werden (z.B. N-Brom-succinimid oder Interhalogene).

Die Erfindung betrifft auch pharmazeutische Präparate, die als Wirkstoff eine oder mehrere Verbindungen der allgemeinen Formel I und geeignete, inerte, übliche pharmazeutische Träger enthalten.

Weitere Einzelheiten der vorliegenden Erfindung sind den nachstehenden Beispielen zu entnehmen, ohne den Schutzumfang auf diese Beispiele einzuschränken.

Beispiel 1

Herstellung des 13-Thia-13,14-dihydro-$PGI_2$-methylesters

A) Herstellung des 5-Jod-13-thia-13,14-dihydro-$PGI_1$-methylesters

Einer Lösung von 102 mg (0,314 mM) 13-Thia-13,14-dihydro-$PGF_{2\alpha}$-methylester in 6 ml wasserfreiem Dichlormethan setzt man unter Rühren 118 mg (0,44 mM) zweimal sublimiertes Jod und danach eine Lösung von 130 mg (0,94 mM) Kaliumcarbonat und 20 ml Wasser zu, rührt das Reaktionsgemisch bei Raumtemperatur 15 - 20 Stunden und gibt danach so lange Natriumthiosulfat zu, bis die Farbe des Jods verschwindet. Die organische Phase trennt man ab, extrahiert die wäßrige Schicht mit 5 ml Dichlormethan, trocknet die vereinigten organischen Phasen über Magnesiumsulfat und engt sie ein. Das als Rückstand erhaltene Öl wird durch Säulenchromatographie gereinigt (Kieselgel G; Elutionsmittel: 7 : 3 Chloroform-Aceton-Gemisch). Es werden 105 mg der im Titel genannten Verbindung erhalten, Ausbeute 65 %.

Nach der Chromatographie enthält das Produkt mehr als 95 % des Exo-Isomers (IIa) und etwa 4 % des Endo-Isomers (IIb).

$R_{f(Exo)}$ = 0,79; $R_{f(Endo)}$ = 0,90.

IR(NaCl): 3400, 1720, 1460, 1360, 1220, 1160, 1050 $cm^{-1}$.

$^1$H-NMR($CDCl_3$): $\delta$ = 0,89 (3H, m), 1,3 (6H, m), 3,68 (3H, s), 4,0 (4H, mc), 4,6 (2H, m).

Ms : $M^+$ 514 (< 1 %), m/e 496 (2, M-18), 369 (3, M-18-I), 255 (22), 223 (54), 205 (6), 191 (18), 173 (7), 148 (21), 143 (20), 111 (37), 108 (59), 91 (14), 81 (71), 54 (100).

B) Herstellung des 13-Thia-13,14-dihydro-$PGI_2$-methylesters

Einer Lösung von 142 mg (0,276 mM) 5-Jod-13-thia-13,14-dihydro-$PGI_1$-methylester in 20 ml wasserfreiem Toluol gibt man unter Argon 0,172 g (1,38 mM) wasserfreies 1,5-Diazabicyclo[4.3.0]non-5-en zu. Das erhaltene Reaktionsgemisch wird unter Argon eine Stunde unter Rückfluß erhitzt, danach abgekühlt, zweimal mit einer 2 %igen wäßrigen Kaliumcarbonatlösung gewaschen, über Magnesiumsulfat getrocknet und unter vermindertem Druck eingeengt. Es werden 106 mg des reinen 13-Thia-13,14-dihydro-$PGI_2$-methylesters erhalten.

$R_f$ = 0,87 (auf einer, mit Triäthylamin behandelten Kieselgelplatte; Laufmittel: festes Kaliumcarbonat enthaltendes, 7 : 3 Chloroform-Aceton-Gemisch).

IR(NaCl): 3350, 1730, 1635, 1440, 1360, 1220, 1150, 1110 $cm^{-1}$.
$^{1}H$-NMR($CDCl_3$-Pyridin-$D_5$): 0,84 (3H, m), 1,3 (6H, m), 3,58 (3H, s) 4,6 (2H, mc).
Ms : $M^+$ 396 (8 %), m/e 368 (13, M-18), 355 (7), 239 (22), 221 (44), 196 (45), 143 (63), 121 (13), 111 (72), 83 (24), 79 (38), 55 (100).

Beispiel 2

Herstellung des 15-Epi-13-thia-13,14-dihydro-$PGI_2$-methylesters

A) Herstellung des 15-Epi-5-jod-13-thia-13,14-dihydro-$PGI_1$-methylesters

Einer Lösung von 159 mg (0,409 mM) 15-Epi-13-thia-13,14-cihydro-$PGF_{2\alpha}$-methylester in 12 ml Dichlormethan wird eine gesättigte wäßrige Lösung von 340 mg (4,1 mM) Natriumbicarbonat zugegeben, danach bei 0°C unter starkem Rühren eine Lösung von 114 mg (0,45 mM) zweimal sublimiertem Jod und 3 ml Dichlormethan zugetropft und das erhaltene Reaktionsgemisch 8 Stunden bei 0°C und 10 Stunden bei Raumtemperatur gerührt. Dem Gemisch gibt man so lange Natriumthiosulfat zu, bis die Jodfarbe verschwindet, trennt die organische Phase ab und extrahiert die wäßrige Schicht mit Mehylenchlorid. Die vereinigten organischen Phasen werden über Magnesiumsulfat getrocknet und unter vermindertem Druck eingeengt. Der Rückstand wird durch Säulenchromatographie gereinigt (Kieselgel G; 7 : 3, Chloroform-Aceton-Gemisch). Ausbeute: 59 %.

Nach der Chromatographie enthält das Produkt mehr als 95 % des Exo-Isomers (IIa) und 4 % des Endo-Isomers (IIb).
$R_{f(Exo)}$ = 0,67; $R_{f(Endo)}$ = 0,78.
IR(NaCl): 3400, 1720, 1460, 1360, 1220, 1160, 1050 $cm^{-1}$.
Ms : $M^+$ 514 (< 1 %), m/e 496 (1, M-18), 369 (4, M-18-I), 255 (20), 223 (58), 205 (7), 191 (17), 173 (7), 148 (21), 143 (22), 111 (38), 108 (60), 91 (14), 81 (71), 54 (100), 45 (60).

B) Herstellung des 15-Epi-13-thia-13,14-dihydro-$PGI_2$-methylesters

Einer Lösung von 94 mg (0,18 mM) 15-Epi-5-jod-13-thia-13,14-dihydro-$PGI_1$-methylester in 10 ml wasserfreiem Toluol wird unter Stickstoff 0,112 g (0,9 mM) wasserfreies 1,5-Diazabicyclo[4.3.0]non-5-en zugegeben, das erhaltene Reaktionsgemisch unter Stickstoff unter Rückfluß

eine Stunde erhitzt, danach auf Raumtemperatur abgekühlt, zweimal mit einer 2 %igen wäßrigen Kaliumcarbonatlösung gewaschen, über Magnesiumsulfat getrocknet und unter vermindertem Druck eingeengt. Es werden 68 mg der im Titel genannten reinen Verbindung erhalten. Ausbeute: 98 %.

$R_f$ = 0,82 (auf einer mit Triäthylamin behandelten Kieselgelplatte; Laufmittel: festes Kaliumcarbonat enthaltendes, 7 : 3 Chloroform-Aceton-Gemisch).

IR(NaCl): 3350, 1720, 1635, 1440, 1360, 1220, 1150 $cm^{-1}$.

$^{1}H$-NMR($CDCl_3$-Pyridin-$D_5$): $\delta$ 0,86 (3H, m), 1,3 (6H, m), 3,62 (3H, s), 4,6 (2H, mc).

Ms : $M^+$ 386 (7), m/e 368 (13, M-18), 355 (6), 239 (21), 221 (35), 196 (28), 195 (12), 143 (43), 138 (12), 137 (10), 131 (12), 121 (29), 115 (15), 111 (50), 109 (14), 97 (16), 95 (22), 83 (21), 81 (20), 79 (33), 67 (19), 59 (18), 55 (100), 43 (25), 41 (53).

## Beispiel 3

### Herstellung des 13-Thia-13,14-dihydro-15-dehydro-$PGI_2$-methylester-äthylen-ketals

#### A) Herstellung des 5-Jod-13-thia-13,14-dihydro-15-dehydro-$PGI_2$-methylester-äthylen-ketals

Einer Lösung von 147 mg (0,341 mM) 13-Thia-13,14-dihydro-15-dehydro-$PGF_{2\alpha}$-methylester-äthylen-ketal und 7 ml Dichlormethan wird eine Lösung von 140 mg (1,02 mM) Kaliumcarbonat und 1 ml Wasser zugegeben und dem erhaltenen Gemisch eine Lösung von 112 mg (0,443 mM) zweimal sublimiertem Jod und 2 ml Dichlormethan zugesetzt. Das Reaktionsgemisch wird bei Raumtemperatur 15 Stunden gerührt und danach eine gesättigte wäßrige Natriumthiosulfatlösung so lange zugegeben bis die Jodfarbe verschwindet. Die organische Phase trennt man ab, extrahiert die wäßrige Schicht mit Dichlormethan, trocknet die vereinigten organischen Phasen über Magnesiumsulfat und engt sie unter vermindertem Druck ein. Der ölige Rückstand wird durch Säulenchromatographie gereinigt (Kieselgel; 7 : 3 Chloroform-Aceton-Gemisch), wobei 120 mg der im Titel genannten Verbindung erhalten werden. Ausbeute 64 %.

Nach der Chromatographie enthält das Produkt mehr als 95 % des Exo-Isomers (IIa).

$R_{f(Exo)}$ = 0,86; $R_{f(Endo)}$ = 0,92.
IR(NaCl): 3370, 1720, 1440, 1410, 1360, 1220, 1180, 1160, 1110, 1060, 1040 $cm^{-1}$.
$^1$H-NMR($CDCl_3$): $\delta$ 0,9 (3H, m) 1,3 (6H, m), 3,68 (3H, s), 4,0 (5H, mc), 4,6 (2H, mc).
Ms : $M^+$ 556 (< 1 %), m/e 497 (2), 496 (8), 495 (23), 494 (100), 463 (4), 438 (4), 386 (7), 368 (7), 254 (8), 223 (11), 209 (8), 189 (8), 144 (4), 143 (35), 127 (7), 115 (10), 111 (38), 99 (36), 91 (13), 81 (28), 79 (34), 71 (27), 55 (40), 44 (51).

B) Herstellung des 13-Thia-13,14-dihydro-15-dehydro-$PGI_2$-methylester-äthylen-ketals

Einer Lösung von 77 mg (0,136 mM) 5-Jod-13-thia-13,14-dihydro-15-dehydro-$PGI_1$-methylester-äthylen-ketal und 10 ml wasserfreiem Toluol gibt man unter Argon 0,084 g (0,679 mM) wasserfreies 1,5-Diazabicyclo[4.3.0]non-5-en zu, wonach man das Reaktionsgemisch unter Argon eine Stunde unter Rückfluß erhitzt. Das erhaltene Gemisch wird auf Raumtemperatur gekühlt, zweimal mit einer 2 %igen gesättigten Kaliumcarbonatlösung gewaschen, über Magnesiumsulfat getrocknet und unter vermindertem Druck eingeengt. Es werden 58 mg der im Titel genannten Verbindung erhalten. Ausbeute: 98 %.
$R_f$ = 0,94 (auf einer mit Triäthylamin behandelten Kieselgelplatte; Laufmittel: festes Kaliumcarbonat enthaltendes, 7 : 3 Chloroform-Aceton-Gemisch).
IR(NaCl): 3350, 1720, 1635, 1440, 1360, 1220, 1150, 1110, 1030 $cm^{-1}$.
$^1$H-NMR($CDCl_3$-Pyridin-$D_5$): $\delta$ 0,86 (3H, m), 1,20 (6H, m), 3,63 (3H, s), 4,1 (4H, mc), 4,6 (2H, m).
Ms : $M^+$ 428 (< 1 %), m/o 397 (1), 386 (3), 385 (7), 384 (26), 366 (5), 353 (8), 285 (6), 271 (18), 270 (20), 253 (17), 252 (8), 239 (21), 238 (9), 237 (8), 222 (6), 221 (22), 209 (7), 199 (15), 196 (30), 195 (18), 189 (12), 167 (16), 143 (100), 141 (7), 135 (8), 133 (7), 123 (8), 121 (14), 111 (52), 99 (20), 95 (23), 91 (9), 83 (19), 81 (25), 79 (28), 73 (16), 71 (24), 55 (48), 43 (60), 41 (38).

0063779

## Beispiel 4

**Herstellung des 13-Thia-13,14-dihydro-$PGF_{2\alpha}$-methylester-13-oxyds und des 15-Epi-13-thia-13,14-dihydro-$PGF_{2\alpha}$-methylester-13-oxyds**

Einer 0,5 molaren wäßrigen Lösung von 0,243 g (1,135 mM) Natriumperjodat tropft man 0,420 g (1,08 mM) eines Gemisches von 13-Thia-13,14-dihydro-$PGF_{2\alpha}$-methylester und 15-Epi-13-thia-13,14-dihydro-$PGF_{2\alpha}$-methylester in 5 ml Methanol gelöst bei 0°C zu, rührt das Reaktionsgemisch bei 0°C 3 Stunden und gibt dem Reaktionsgemisch so viel Wasser zu, daß das ausgeschiedene kristalline Natriumjodat in Lösung geht. Die wäßrige Lösung wird mit 20 ml Dichlormethan extrahiert, der Extrakt über Magnesiumsulfat getrocknet und der ölige Rückstand durch Säulenchromatographie gereinigt. Die Isomere werden voneinander getrennt (Kieselgel G; 7 : 3 Chloroform-Aceton-Gemisch). Es werden 0,152 mg des 13-Thia-13,14-dihydro-$PGF_{2\alpha}$-methylester-13-oxyds und 0,139 g des 15-Epi-13-thia-13,14-dihydro-$PGF_{2\alpha}$-methylester-13-oxyds erhalten. Gesamtausbeute: 0,291 g (66,5 %).

$R_f$ = 0,27 (Chloroform-Aceton, 7 : 3);

$R_{f(15-Epi)}$ = 0,22 (Chloroform-Aceton, 7 : 3).

Ms : $M^+$ 404 (7), m/e 389 (2), 387 (5), 373 (5), 333 (10), 285 (12), 284 (20), 242 (15), 241 (10), 224 (30), 222 (18), 210 (22), 204 (20), 185 (40), 173 (25), 163 (40), 147 (30), 86 (70), 45 (100).

Ms-15-epi : $M^+$ 404 (6), m/e 389 (2), 387 (6), 373 (4), 333 (10), 285 (12), 284 (16), 242 (16), 241 (10), 224 (30), 222 (16), 210 (22), 204 (20), 185 (40), 173 (23), 163 (36), 147 (34), 86 (62), 45 (100).

## Beispiel 5

**Herstellung des 13-Thia-13,14-dihydro-$PGI_2$-Natriumsalzes**

71 mg (0,138 mM) 13-Thia-13,14-dihydro-$PGI_2$-methylester werden in 0,3 ml Methanol gelöst, wonach man der Lösung 2 ml einer 0,1 n wäßriger Natriumhydroxydlösung zugibt und das Reaktionsgemisch 24 Stunden bei Raumtemperatur rührt. Nach Lyophilisierung der Lösung werden 76 mg des weißen, im Titel genannten Natriumsalzes erhalten.

## Patentansprüche

1. Optisch aktive oder racemische 13-Thia-prostacyclin-Derivate der allgemeinen Formel I

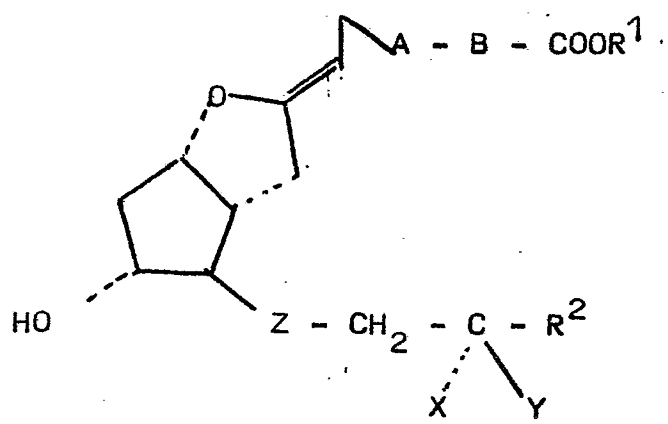

(I) ,

worin

$R^1$ Wasserstoff, ein Äquivalent eines pharmazeutisch verträglichen Kations oder geradkettiges oder verzweigtes $C_{1-5}$-Alkyl,

$R^2$ geradkettiges oder verzweigtes $C_{4-9}$-Alkyl oder monosubstituiertes Phenoxymethyl,

-A-B- $-CH_2-CH_2-$, Z- oder E- -CH=CH-, oder -C≡C-,

eines der Symbole X und Y Wasserstoff, Methyl oder Äthyl und das andere Hydroxy, Tetrahydropyranyloxy, 1-Äthoxy-äthoxy oder Tri-$C_{1-5}$-alkylsilyloxy bedeuten oder

X und Y gemeinsam $-O-CH_2-CH_2-O-$ bilden und

Z Schwefel oder -SO- ist.

2. Verbindungen der allgemeinen Formel II

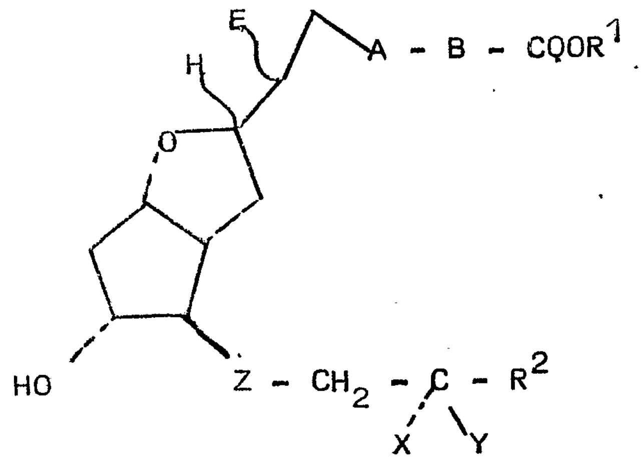

(II) ,

worin

$R^1$, $R^2$, -A-B-, X und Y und Z die oben angegebene Bedeutung haben, und E Halogen ist.

3. Methylester von
5-Jod-13-thia-13,14-dihydro-$PGI_1$,
15-Epi-5-jod-13-thia-13,14-dihydro-$PGI_1$,
13-Thia-13.14-dihydro-$PGI_2$,
15-Epi-13-thia-13,14-dihydro-$PGI_2$,
13-Thia-13,14-dihydro-15-dehydro-$PGI_2$,
13-Thia-13,14-dihydro-$PGF_{2\alpha}$-13-oxyd und
15-Epi-13-thia-13,14-dihydro-$PGF_{2\alpha}$-13-oxyd.

4. Methylester-äthylen-ketal von
5-Jod-13-thia-13,14-dihydro-15-dehydro-$PGI_1$ und
13-Thia-13,14-dihydro-15-dehydro-$PGI_2$.

5. Natriumsalz von 13-Thia-13,14-dihydro-$PGI_2$.

6. Verfahren zur Herstellung von optisch aktiven oder racemischen Verbindungen der allgemeinen Formel I

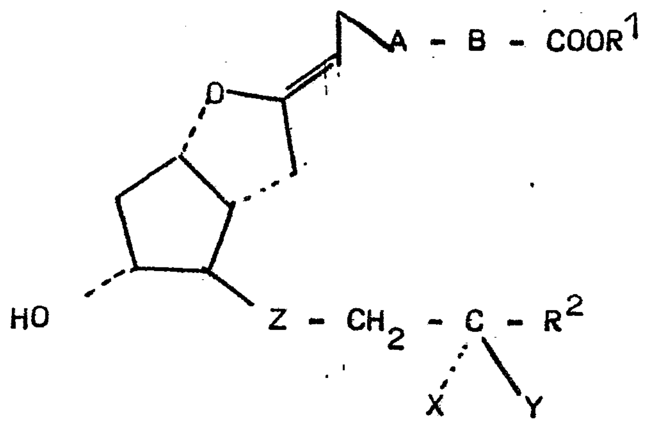

(I) ,

worin

$R^1$ Wasserstoff, ein Äquivalent eines pharmazeutisch verträglichen Kations oder geradkettiges oder verzweigtes $C_{1-5}$-Alkyl,

$R^2$ geradkettiges oder verzweigtes $C_{4-9}$-Alkyl oder monosubstituiertes Phenoxymethyl,

-A-B- $-CH_2-CH_2-$, Z- oder E- -CH=CH-, oder -C≡C-,

eines der Symbole X und Y Wasserstoff, Methyl oder Äthyl und das andere Hydroxy, Tetrahydropyranyloxy, 1-Äthoxy-äthoxy oder Tri-$C_{1-5}$-alkylsilyloxy bedeuten oder

X und Y gemeinsam $-O-CH_2-CH_2-O-$ bilden und

Z Schwefel oder -SO- ist,

dadurch gekennzeichnet, daß man ein 13-Thia-prostaglandin-Derivat der allgemeinen Formel III

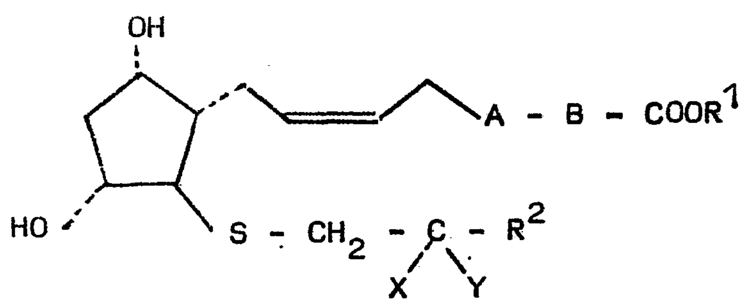

(III),

worin

$R^1$, $R^2$, -A-B-, X und Y die oben angegebene Bedeutung haben, gewünschtenfalls nach Oxydation zum entsprechenden 13-Oxyd-Derivat in Gegenwart einer Base mit einem Halogenierungsmittel umsetzt, das erhaltene 5-Halogen-13-thia-prostacyclin-Derivat der allgemeinen Formel II

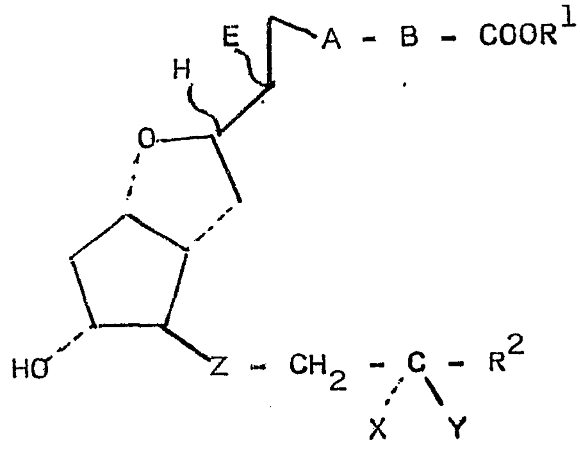

(II),

worin

$R^1$, $R^2$, -A-B-, X, Y und Z die oben angegebene Bedeutung haben und E Halogen ist - falls E Brom ist, nach Abtrennung des Endo-Isomers bzw. falls E Jod ist, in Form des Isomerengemisches - mit einer organischen oder anorganischen Base behandelt und das erhaltene 13-Thia-prostacyclin-Derivat der allgemeinen Formel I gewünschtenfalls - wenn X und/oder Y eine Schutzgruppe tragen - durch Hydrolyse von der Schutzgruppe befreit und/oder in ein Salz überführt oder aus einem Salz freisetzt.

7. Verfahren nach Anspruch 6, dadurch gekennzeichnet, daß man die Oxydation zum 13-Oxyd-Derivat mit Natriumperjodat durchführt.

8. Verfahren nach Anspruch 6 oder 7, dadurch gekennzeichnet, daß man als Halogenierungsmittel ein Jodierungsmittel, vorzugsweise elementares Jod, verwendet.

9. Verfahren nach Anspruch 6, 7 oder 8, dadurch gekennzeichnet, daß man die Halogenierung in Gegenwart von Alkalimetallcarbonaten durchführt.

10. Verfahren nach Anspruch 6, 7, 8 oder 9, dadurch gekennzeichnet, daß man die Halogenierung in einem mäßig polaren aprotischen Lösungsmittel durchführt.

11. Verfahren nach Anspruch 6, 7, 8, 9 oder 10, dadurch gekennzeichnet, daß man die Dehydrohalogenierung der 5-Halogen-13-thia-prostacyclin-Derivate der allgemeinen Formel II mit 1,5-Diaza-bicyclo[4.3.0]-non-5-en durchführt.

12. Verfahren nach Anspruch 6, 7, 8, 9, 10 oder 11, dadurch gekennzeichnet, daß man die Dehydrohalogenierung in Toluol beim Siedepunkt des Reaktionsgemisches durchführt.

13. Pharmazeutische Präparate die als Wirkstoff ein oder mehrere 13-Thia-prostacyclin-Derivate der Ansprüche 1 bis 5 enthalten.

Europäisches Patentamt

**EUROPÄISCHER RECHERCHENBERICHT**

0063779

Nummer der Anmeldung

EP 82 10 3330

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | BE-A- 828 925 (MERCK PATENT)<br>* Ansprüche *<br>--- | 1,2,6, 10-13 | C 07 D 307/935<br>A 61 K 31/34 |
| Y | CHEMISTRY, BIOCHEMISTRY & PHARMACOLOGICAL ACTIVITY OF PROSTANOIDS, Including the proceedings of a symposium on the chemistry and biochemistry of prostanoids held at the university of Salford, G.B., 10.-14. Juli 1978, Seiten 286-312,<br>K.C. NICOLAOU et al.: "Synthesis and biological properties of prostacyclins and prostaglandin endoperoxide analogs" * Seiten 289-295 *<br>----- | 1,2,6, 10-13 | |

| RECHERCHIERTE SACHGEBIETE (Int. Cl. 3) |
|---|
| C 07 D 307/00 |

Der vorliegende Recherchenbericht wurde für alle Patentansprüche erstellt.

| Recherchenort | Abschlußdatum der Recherche | Prüfer |
|---|---|---|
| DEN HAAG | 23-07-1982 | BERTE M.J. |

KATEGORIE DER GENANNTEN DOKUMENTEN
- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument

EPA Form 1503 03.82